(19) Europäisches Patentamt
European Patent Office
Office européen des brevets

(11) **EP 3 041 603 B1**

(12) **EUROPÄISCHE PATENTSCHRIFT**

(45) Veröffentlichungstag und Bekanntmachung des Hinweises auf die Patenterteilung:
**29.09.2021   Patentblatt 2021/39**

(21) Anmeldenummer: **14755605.4**

(22) Anmeldetag: **11.08.2014**

(51) Int Cl.:
**B01J 19/30** *(2006.01)*      **B01D 3/00** *(2006.01)*
**C07C 51/25** *(2006.01)*      **B01D 3/14** *(2006.01)*
**B01D 3/16** *(2006.01)*       **C07C 51/48** *(2006.01)*

(86) Internationale Anmeldenummer:
**PCT/EP2014/067137**

(87) Internationale Veröffentlichungsnummer:
**WO 2015/028293 (05.03.2015 Gazette 2015/09)**

(54) **VERFAHREN ZUR HERSTELLUNG VON ACRYLSÄURE**

METHOD FOR PRODUCING ACRYLIC ACID

PROCÉDÉ DE PRODUCTION D'ACIDE ACRYLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.09.2013   DE 102013217386**

(43) Veröffentlichungstag der Anmeldung:
**13.07.2016   Patentblatt 2016/28**

(73) Patentinhaber: **Evonik Operations GmbH**
**45128 Essen (DE)**

(72) Erfinder:
• **MOSLER, Jürgen**
  **45772 Marl (DE)**
• **PSIORZ, Christian**
  **40883 Ratingen (DE)**
• **KUPPINGER, Franz-Felix**
  **45768 Marl (DE)**
• **SCHRIEWER, Raphael**
  **48366 Laer (DE)**
• **BRUHNS, Arnd**
  **33829 Borgholzhausen (DE)**
• **RIX, Armin**
  **45770 Marl (DE)**
• **KREIS, Peter**
  **44227 Dortmund (DE)**
• **MEIER, Ralf**
  **44265 Dortmund (DE)**
• **ELDER, James Edward**
  **Friendswood, Texas 77546 (US)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A2- 0 130 745      EP-A2- 0 982 289**
**US-B1- 6 293 528**

**Beschreibung**

Gegenstand der vorliegenden Erfindung ist ein Verfahren zur Herstellung von Acrylsäure, welches einen Schritt der Oxidation von Acrolein aufweist, wobei das bei dieser Oxidation erhaltene Reaktionsgemisch in einer erfindungsgemäßen Apparatur mit Wasser als Quenchmittel in Kontakt gebracht wird.

Acrylsäure (Prop-2-ensäure) ist ein Grundbaustein für die Herstellung von vornehmlich in Hygieneprodukten eingesetzten Superabsorbern sowie Rohstoff für die komplette Acrylatchemie. Acrylsäure wird im großtechnischen Maßstab durch katalytische Dampfphasenreaktion von Propen mit Sauerstoff erhalten. Dabei entstehen neben der gewünschten Acrylsäure auch unerwünschte Begleitprodukte, die bei der Weiterverarbeitung der Acrylsäure hinderlich sind. Bei diesen Begleitprodukten handelt es sich im Wesentlichen um Essigsäure, Aldehyde, Dialdehyde, Dicarbonsäuren und Wasser. Ein wichtiger Schritt bei der Herstellung von Acrylsäure (AS) besteht demnach darin, die aus der Dampfphasenreaktion stammende, acrylsäurehaltige Zusammensetzung von den unerwünschten Begleitprodukten zu reinigen, sodass reine Acrylsäure übrig bleibt. Die vorliegende Erfindung befasst sich mit einem Teilaspekt dieser Aufreinigung.

Die Herstellung von Acrylsäure erfolgt üblicherweise klassisch chemisch über die Oxidation von Propen zum Acrolein und anschließende weitere Oxidation zur Acrylsäure. Der EP 1319648 A1 kann ein solches Verfahren entnommen werden, bei dem die Herstellung von Acrylsäure (AS) durch katalytische Dampfphasenoxidation von Propylen in zwei Schritten erfolgt. Das Verfahren zur Herstellung von AS durch die zweistufige (die erste katalytische Oxidationsreaktionsstufe für die Umsetzung von Propylen hauptsächlich zu Acrolein und die zweite katalytische Oxidationsreaktionsstufe für die Umsetzung von Acrolein zu AS) katalytische Dampfphasenoxidation von Propylen unter Verwendung von molekularem Sauerstoff ist bereits bekannt und wird in industriellem Maßstab seit vielen Jahrzehnten durchgeführt. Aus vielen Gründen (Entflammbarkeits-Grenzen für das Gemisch Propylen/Luft und hohe Reaktionswärme) ist es notwendig, die Reaktionsgase durch Inertgase (zum Beispiel Wasserdampf, $N_2$, $CO_2$) zu verdünnen. Ein typisches Verfahren für die industrielle Herstellung sieht wie folgt aus: Eine Mischung aus Propylen, Luft und Dampf wird einem ersten Oxidationsreaktor zugeführt und das Propylen wird im ersten Schritt hauptsächlich zu Acrolein und in geringen Mengen zu AS umgesetzt. Das Produkt wird ohne Auftrennung einem zweiten Oxidationsreaktor zugeführt. Frische Luft und Wasserdampf können, wenn dieses für die nachfolgende Oxidationsreaktion im zweiten Oxidationsschritt erforderlich ist, am Einlass des zweiten Oxidationsreaktors zugesetzt werden.

Für die Trennung der gasförmigen AS vom Wasserdampf in dem etwa 180°C warmen Abfluss aus den Oxidationsreaktoren werden zwei Trennverfahren verwendet: 1. Absorption der gasförmigen AS in einem hochsiedenden, hydrophoben, aromatischen Lösungsmittel bei Temperaturen, unter denen das Prozesswasser im Prozess-Abluftgas, welches einen Absorptionsturm oben verlässt, verbleibt (siehe zum Beispiel DE 43 08 087 BASF, 15.09.94) 2. Absorption der AS in Wasser bei gleichzeitigem Quenchen auf niedrige Temperaturen in einem Quenchturm/Kollektor in einer solchen Weise, dass nahezu das gesamte verdampfte Prozesswasser im 180°C-Abfluss aus dem zweiten Reaktor kondensiert (siehe zum Beispiel DE 30 42 468/Mitsubishi Chem. Corp. (MCC), 11.11.80). Während bei der ersten Verfahrensweise die AS und die hochsiedenden Produkte in vielen Destillationsschritten getrennt werden, wird bei der zweiten Verfahrensweise das Wasser in der wässrigen AS nach dem Quenchen in einer nachfolgenden azeotropen Destillation abgetrennt, um eine Roh-AS zu erhalten, aus der AS mit hoher Reinheit für die Herstellung von AS-Copolymeren oder verschiedene AS-Ester hergestellt werden können.

Bei der zweiten Verfahrensweise, hat die Handhabung des Prozesswassers, welches aus dem bei der Oxidation gebildeten Wasser und dem Wasser für die Verdünnung, welche erforderlich ist, um außerhalb Entflammbarkeits-Grenze zu sein, besteht, einen beachtlichen Einfluss auf die Wirtschaftlichkeit des Verfahrens.

Wenn das AS beinhaltende Produktgas, welches am Auslass des zweiten Oxidationsreaktors erhalten wird, in den Quenchturm eingebracht wird, um AS als wässrige Lösung zu erhalten, dann verlässt das resultierende Prozess-Abluftgas, welches nicht reagiertes Propylen und andere niedrig siedende organische Materialien beinhaltet, oben den Quenchturm und muss zum Beispiel in einem Verbrennungsofen behandelt werden, so dass keine oder nahezu keine organischen Verbindungen in die Luft emittiert werden (ein Durchlaufprozess mit einem Durchgang).

Es ist auch möglich, dass ein Teil des Prozess-Abluftgases rezykliert und dem Propylen/Luft/Wasserdampf-Strom am Einlass der ersten Reaktionsstufe zugesetzt wird (Kreisgas-Strömungsprozess), was eine durchaus übliche Vorgehensweise insbesondere für den Fall darstellt, dass das Verfahren mit teilweiser Umsetzung des Propylens betrieben wird.

In DE 10 2010 028 781 A1 wird die weitere Auftrennung eines in einem Quenchturm erhaltenen wässrigen Acrylsäure aufweisenden Gemisches mit Hilfe einer Destillationskolonne, die einen Seitenabzug aufweist beschrieben.

In US 7189872 wird ein Verfahren zur Herstellung von Acrylsäure beschrieben, bei dem ein Quenchturm eingesetzt wird, um Acrylsäure aus dem gasförmigen Produktgemisch abzutrennen. Dieses wässrige Gemisch wird anschließen mittels azeotroper Destillation in eine gasförmige, Wasser aufweisende Phase und eine flüssige, Acrylsäure aufweisende Phase, getrennt.

EP 0982289 A2 offenbart eine Apparatur und ein Verfahren zur Herstellung Acrylsäure, wobei das Quenchmittel Wasser ist.

US 6293528 B1 offenbart eine Apparatur mit Lochboden.

Bei der Acrylsäure-Herstellung durch Oxidation von Propen mit Luftsauerstoff in geringen Konzentrationen entstehen auch Nebenkomponenten, wie z. B. Phthalsäure, Therephthalsäure oder Isophthalsäure. Diese Stoffe stellen Schwersiederverbindungen dar und sind nur schlecht in Wasser löslich.

Bei der Verwendung eines Quenchturms zur Aufbereitung des gasförmigen Produktgemisches aus dem letzten Oxidationsreaktor, fallen die genannten Nebenkomponenten im Sumpf mit der wässrigen AS an. Die Konzentration der Nebenkomponenten (Summe aus Phthalsäure, Therephthalsäure oder Isophthalsäure) beträgt 0,024 bis 0,030 Gew.-% bezogen auf die im Sumpf des Quenchturms erhaltene Zusammensetzung.

Der Quenchturm wird häufig so ausgeführt, dass er sowohl das gasförmige Reaktionsgemisch kühlen und kondensieren, aber auch den Verlust von Acrylsäure im Kopf des Turms minimieren kann. Der Quenchturm ist deshalb üblicherweise mit Destillationsböden ausgerüstet, wie z.B. Glockenböden oder Tunnel- bzw. Thormannböden. Auch Ventilböden sieht man im Einsatz.

Will man nun den Durchsatz durch so einen Quenchturm signifikant erhöhen oder den Kopfverlust des Zielproduktes reduzieren, so ist es bei Destillationsböden häufig üblich die Böden durch Packungen, insbesondere strukturierte Packungen zu ersetzen.

Dabei wurde jedoch gefunden, dass jetzt nur noch ein Teil der Schwersiederverbindungen im Sumpf des Quenchturms ausgetragen wird und der andere Teil als Aerosol bzw. Desublimat bis in den Kopf der Kolonne gelangt und dort und ggf. in nachgeschalteten Apparaten zu Verstopfungen führt, so dass die Anlage nach nur einer geringen Laufzeit abgestellt werden muss, um Reinigungsarbeiten durchzuführen. Im Sumpf der Kolonne werden die oben genannten Nebenkomponenten nur noch in einer Konzentration von < 0,02 Gew-%, bezogen auf die Zusammensetzung im Sumpf des Quenchturms nachgewiesen.

Aufgabe der vorliegenden Erfindung war es deshalb die Laufzeit des Quenchturms wieder zu verbessern, ohne dass der Durchsatz durch den Quenchturm verringert oder der Kopfverlust des Zielprodukts erhöht wird.

Überraschenderweise wurde gefunden, dass diese Aufgabe gelöst werden kann, in dem im Quenchturm zwischen Zulauf und einer zwischen Quenchmittelzulauf und Zulauf vorhandenen Packung, mindestens ein Mittel vorgesehen wird, welches die Ausbildung einer flüssigen Schicht mit einer Höhe von mindestens 1 cm gewährleistet.

Wesentliches Element der vorliegenden Erfindung ist deshalb eine Apparatur umfassend eine Zuführung die unterhalb der Mitte der Apparatur angeordnet ist, einen Quenchmittelzulauf, der oberhalb der Zuführung angeordnet ist, einen Ablauf, der unterhalb der Zuführung angeordnet ist, einen Abzug, der oberhalb des Quenchmittelzulaufs angeordnet ist, wobei die Apparatur zwischen Quenchmittelzulauf und Zuführung einen Bereich aufweist, der mit einer oder mehreren Packungen versehen ist, dadurch gekennzeichnet, dass zwischen dem Quenchmittelzulauf und einer zwischen Quenchmittelzulauf und Zuführung vorhandenen Packung, mindestens ein Mittel vorhanden ist, welches die Ausbildung einer flüssigen Schicht mit einer Höhe von mindestens 1 cm gewährleistet. Bei dem Mittel handelt es sich erfindungsgemäß um einen Siebboden oder um einen Wellenboden (ripple tray).

Gegenstand der vorliegenden Erfindung ist ein Verfahren zur Herstellung von Acrylsäure, welches einen Schritt der Oxidation von Acrolein aufweist, wobei das bei dieser Oxidation erhaltene Reaktionsgemisch in genannter Apparatur mit Wasser als Quenchmittel in Kontakt gebracht wird und wobei die flüssige Schicht in der Apparatur so angeordnet ist, dass gasförmige Stoffe, die durch die Zuführung in die Apparatur gelangen, durch die flüssige Schicht hindurchtreten, um in den Kopf der Apparatur zu gelangen. Als Wasser kann z. B. vollentsalztes Wasser oder Prozesswasser verwendet werden, welches ggf. Essigsäure, Propionsäure und geringe Mengen Acrylsäure enthalten kann, aus einer nachfolgenden Wasserabtrennkolonne.

Das erfindungsgemäße Verfahren hat den Vorteil, dass bei annähernd gleichem Durchsatz und ohne Erhöhung der Kopfverluste an Zielprodukt im Quenchturm die Laufzeit (Standzeit) erhöht wird.

Ein weiterer Vorteil des erfindungsgemäßen Verfahrens liegt darin, dass ein Überschleppen bzw. Austragen von Dicarbonsäuren mit dem Kopfstrom vermieden oder zumindest reduziert wird, so dass die Dicarbonsäuren die weitere Aufarbeitung des Kopfprodukts nicht stören.

Ein weiterer Vorteil des erfindungsgemäßen Verfahrens ist, dass durch die Verwendung eines Siebbodens Verstopfungen, wie sie bei der Verwendung anderer Böden auftreten, wie z. B. bei der Verwendung von Tunnelböden, vermieden werden können, woraus wiederum eine längere Laufzeit bzw. längere Wartungsintervalle resultieren. Die notwendigen Betriebsunterbrechungen werden durch die Nutzung des erfindungsgemäßen Verfahrens somit deutlich reduziert.

Die erfindungsgemäßen Gegenstände werden nachfolgend beispielhaft beschrieben. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Die Apparatur, umfasst eine Zuführung, die unterhalb der Mitte der Apparatur angeordnet ist, einen Quenchmittelzulauf, der oberhalb der Zuführung angeordnet ist, einen Ablauf, der unterhalb der Zuführung angeordnet ist, einen Abzug, der oberhalb des Quenchmittelzulaufs angeordnet ist, wobei die Apparatur zwischen Quenchmittelzulauf und Zuführung einen Bereich aufweist, der mit einer oder mehreren Packungen versehen ist, zeichnet sich dadurch aus, dass zwischen dem Quenchmittelzulauf und einer zwischen Quenchmittelzulauf und Zuführung vorhandenen Packung, mindestens ein Mittel vorhanden ist, welches die Ausbildung einer flüssigen Schicht mit einer Höhe von mindestens 1 cm, vorzugsweise mit einer Höhe von 2 bis 10 cm, bevorzugt mit einer Höhe von 3 bis 5 cm, gewährleistet, wobei die flüssige Schicht erfindungsgemäß in der Apparatur so angeordnet ist, dass gasförmige Stoffe, die durch die Zuführung in die Apparatur gelangen, durch die flüssige Schicht hindurchtreten müssen, um in den Kopf der Apparatur zu gelangen, weshalb die flüssige Schicht auch als Sprudelschicht bezeichnet wird.

Erfindungsgemäße Mittel sind Siebböden oder Wellenböden (Ripple-Tray). Bei einer Gestaltung der Mittel in der Weise, dass die aufströmenden Gase vor dem Hindurchtreten durch die flüssige Schicht über einen längere Zeit mit dem Mittel Kontakt haben, wie dies z. B. bei Glockenböden, Tunnelböden oder Thormannböden der Fall ist, kann es zu Ablagerungen von Disäuren an den Oberflächen dieser Mittel kommen. Es ist deshalb besonders vorteilhaft, als Mittel solche zu nehmen, bei denen die aufströmenden Gase ohne langen Kontakt mit dem Mittel durch die flüssige Schicht hindurchtreten, wie dies z. B. bei Wellenböden oder Siebböden der Fall ist, weshalb diese als Mittel erfindungsgemäß sind.

Vorzugsweise sind in der Apparatur von 1 bis 5, bevorzugt 2 oder 3, besonders bevorzugt 3 der Mittel zwischen dem Quenchmittelzulauf und einer zwischen Quenchmittelzulauf und Zuführung vorhandenen Packung vorhanden.

Als Packung(en) können in der Apparatur alle Arten von geeigneten Packungen vorhanden sein. Vorzugsweise ist die Packung bzw. sind die Packungen strukturierte Packungen oder Gewebepackungen, bevorzugt strukturierte Packungen. Geeignete Packungen werden u.a. von Sulzer unter den Handelsbezeichnungen Mellapak, von Raschig unter der Handelsbezeichnung Super-Pak, von Koch-Glitsch unter der Handelsbezeichnung Flexi-Pak sowie von Montz unter den Handelsbezeichnungen Montz-Pak vertrieben.

Die Packung bzw. die Packungen in der Apparatur weisen vorzugsweise eine Höhe der Packung auf, die 40 bis 85 %, bevorzugt 45 bis kleiner 55 % der inneren Höhe der Apparatur beträgt. Sind mehr als eine Packung in der Apparatur vorhanden, so ergibt sich die Höhe der Packung aus der Summe der Höhen der einzelnen Packungen. Die innere Höhe der Apparatur wird durch Bestimmen der Strecke von höchstem Punkt im Kopf der Apparatur zu niedrigstem Punkt im Sumpf der Apparatur ermittelt.

Es kann vorteilhaft sein, wenn zwischen dem untersten Mittel oberhalb der Packung und der obersten Packung ein Verteiler vorhanden ist, der die vom untersten Mittel abfließende flüssige Phase auf der Packung möglichst gleichmäßig verteilt. Ist ein solcher Verteiler vorhanden hat es sich außerdem als vorteilhaft erwiesen, wenn zwischen unterstem Mittel und Verteiler ein Sammler angeordnet ist, der die vom untersten Mittel abfließende flüssige Phase sammelt und dem Verteiler zuführt.

Das erfindungsgemäße Verfahren zur Herstellung von Acrylsäure, welches einen Schritt der Oxidation von Acrolein aufweist, zeichnet sich dadurch aus, dass das bei dieser Oxidation erhaltene Reaktionsgemisch in einer wie oben beschrieben Apparatur mit Wasser als Quenchmittel in Kontakt gebracht wird. Als Quenchmittel kann Wasser eingesetzt werden, wie oben beschrieben.

Als Reaktionsgemisch kann jegliches, Acrylsäure aufweisendes Gemisch, welches vorzugsweise gasförmig ist, eingesetzt werden. Geeignete Gemische können z. B. durch Verfahren zur Herstellung von Acrylsäure erhalten werden, wie sie in DE 19740252, EP 0092097, EP 0058927 oder EP 0608838 beschrieben werden. In Bezug auf die Herstellung von Acrylsäure wird ausdrücklich auf die in den genannten Dokumenten gemachten Angaben Bezug genommen.

Das Verhältnis der Volumenströme von Quenchmittel, welches der Apparatur zugeführt wird, zu Reaktionsgemisch, welches der Apparatur zugeführt wird, beträgt vorzugsweise von 1 zu 2 bis 1 zu 20, bevorzugt von 1 zu 5 bis 1 zu 10 und besonders bevorzugt von 1 zu 7 bis 1 zu 8.

Der Apparatur wird pro Stunde vorzugsweise von 210 kg bis 380 kg, bevorzugt von 230 kg bis 260 kg Reaktionsgemisch pro m$^3$ Apparaturvolumen zugeführt. Das Apparaturvolumen ist das Volumen der Apparatur minus dem Volumen der Einbauten. Das Apparaturvolumen kann durch Füllen der Apparatur mit einer Flüssigkeit bestimmt werden.

Die Temperatur der flüssigen Phase auf dem Mittel beträgt vorzugsweise von 50 °C bis 100 °C, bevorzugt von 60 °C bis 90 °C und besonders bevorzugt von 70 °C bis 80 °C. Die Temperatur kann durch Variation der Volumenströme sowie durch die der Apparatur zugeführte Wärmeenergie eingestellt werden.

In dem erfindungsgemäßen Verfahren wird die Apparatur vorzugsweise so betrieben, dass die Druckdifferenz zwischen dem Druck, gemessen am Kopf der Apparatur und dem Druck, gemessen im Gasraum oberhalb der flüssigen Phase im Sumpf der Apparatur von 10 mbar bis 60 mbar, bevorzugt von 30 mbar bis 40 mbar beträgt.

Mittels des erfindungsgemäßen Verfahrens sind Zusammensetzungen (Produktgemische) erhältlich, die überwiegend Acrylsäure und Wasser enthalten. Vorzugsweise wird die Zusammensetzung als Sumpfprodukt (der erfindungsgemäß eingesetzten Apparatur) in dem erfindungsgemäßen Verfahren erhalten. Die Zusammensetzung enthält vorzugsweise Acrylsäure, Wasser, Phthalsäure, Terephthalsäure und Isophthalsäure, wobei der Anteil der Summe aus Phthalsäure, Terephthalsäure und Isophthalsäure vorzugsweise größer 0,05 Gew.-%, bevorzugt von 0,05 bis 1 Gew.-% und besonders bevorzugt von 0,052 bis 0,075 Gew.-% bezogen auf die Zusammensetzung beträgt. Die Summe aus Wasser und Acrylsäure beträgt vorzugsweise größer 90 Gew.-%, bevorzugt größer 95 Gew.-% bezogen auf die Zusammensetzung. Der Anteil an Acrylsäure an der Zusammensetzung beträgt vorzugsweise von 50 bis 65 Gew.-%, bevorzugt 53 bis 62 Gew.-% bezogen auf die Zusammensetzung. Der bevorzugte Anteil an Wasser ergibt sich aus den für die Summe aus Acrylsäure und Wasser angegebenen Werten entsprechend. Besonders bevorzugte Zusammensetzungen weisen einen Anteil der Summe aus Phthalsäure, Terephthalsäure und Isophthalsäure von vorzugsweise größer 0,05 Gew.-%, bevorzugt von 0,05 bis 1 Gew.-% und besonders bevorzugt von 0,052 bis 0,075 Gew.-% bezogen auf die Zusammensetzung und einen Anteil der Summe aus Wasser und Acrylsäure von vorzugsweise größer 90 Gew.-%, bevorzugt größer 95 Gew.-% bezogen auf die Zusammensetzung. Als weitere Komponenten können die Zusammensetzungen Essigsäure, Maleinsäure, Hydrochinon, Di-Acrylsäure, Formaldehyd und Propionsäure aufweisen. Besonders bevorzugte Zusammensetzungen weisen von 2 bis 3 Gew.-% Essigsäure, von 0,1 bis 0,3 Gew.-% Maleinsäure, von 0,03 bis 0,1 Gew.-% Hydrochinon, von 0,15 bis 0,25 Gew.-% Di-Acrylsäure, von 0,5 bis 1,25 Gew.-% Formaldehyd und von 0,01 bis 0,05 Gew.-% Propionsäure, jeweils bezogen auf die Zusammensetzung auf.

Die erhaltenen Zusammensetzungen (Produktgemische) können einer weiteren Aufarbeitung zugeführt werden. Insbesondere kann die Aufarbeitung der Zusammensetzung wie in DE 10 2010 028 781 A1 oder US 7189872 beschrieben, auf welche ausdrücklich Bezug genommen wird, erfolgen.

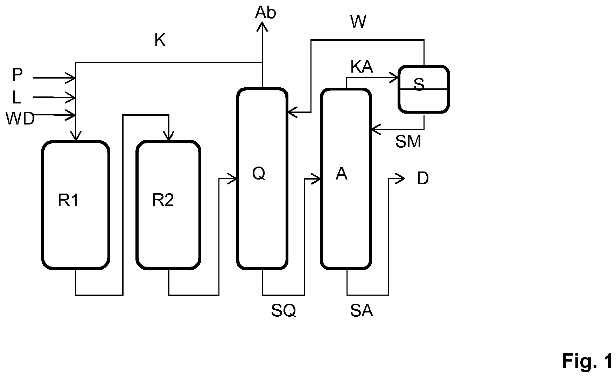

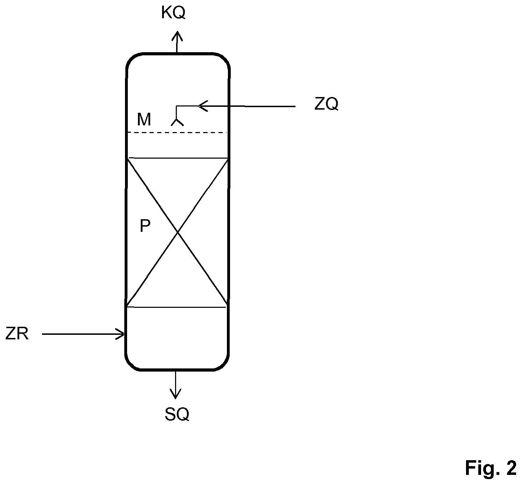

Die vorliegende Erfindung wird anhand der Figuren Fig. 1 und Fig. 2 näher erläutert.

Fig. 1 zeigt schematisch (Übliche Apparaturen wie z.B. Schieber, Hähne, Pumpen oder Wärmetauscher wurden der besseren Übersichtlichkeit halber nicht dargestellt) eine Anlage zur Herstellung von Acrylsäure aus Propen. In den ersten Oxidationsreaktor R1 werden Propen P, Wasserdampf (WD), und Luft (L) geleitet. Das Reaktionsprodukt, welches Acrolein enthält, wird in einen zweiten Oxidationsreaktor R2 geleitet, in dem das Acrolein zur Acrylsäure oxidiert wird. Das gasförmige Reaktionsprodukt aus Reaktor R2 wird seitlich in den Quenchturm Q gefahren, in den von oben Wasser eingeleitet wird. Das gasförmige Kopfprodukt aus dem Quenchturm kann teilweise als Abgas einer weitere Aufarbeitung oder Entsorgung zugeführt und teilweise als Kreisgas in den Reaktor R1 zurückgefahren werden. Das Sumpfprodukt des Quenchturms SQ wird seitlich in Destillationskolonne A überführt, in der eine Schleppmittel-/AzeotropDestillation durchgeführt wird. Das Kopfprodukt KA wird kondensiert und in einen Abscheider S überführt, in dem das Kopfprodukt in eine wässrige Phase und eine das Schleppmittel aufweisende Phase getrennt wird. Das Schleppmittel SM wird in die Destillationskolonne A oberhalb der Einspeisung des Sumpfprodukts SQ zurückgeführt. Die im Abscheider S erhaltene wässrige Phase W wird in den Quenchturm oberhalb der Einspeisung des Reaktionsproduktes aus dem Reaktor R2 zurückgeführt. Das Sumpfprodukt SA der Kolonne A kann einer weitere Aufbereitung D zugeführt werden.

Fig. 2 zeigt schematisch einen Aufbau einer Apparatur (eines Quenchturms): Die Apparatur ist mit einer Zuführung für das Reaktionsgemisch ZR, einem Zulauf für das Quenchmittel ZQ, einem Ablauf im Sumpf SQ und einem Abzug für das Kopfprodukt KQ versehen. Im inneren ist eine Packung P vorgesehen, über dem ein Mittel M, welches die Ausbildung einer flüssigen Schicht mit einer Höhe von mindestens 1 cm gewährleistet, angeordnet ist.

In den nachfolgend aufgeführten Beispielen wird die vorliegende Erfindung beispielhaft beschrieben.

**Beispiele**

**Messmethode:**

Die Bestimmung des Anteils an Phthalsäure, Isophthalsäure und Terephthalsäure im Sumpfprodukt der Apparatur erfolgte mittels GC/MS als Dimethylester. Dazu wurden die Proben zur Veresterung zunächst mit Diazomethan versetzt. Die so präparierten Proben wurden dem GC/MS zugeführt (Nachfolgend beschrieben für Terephthalsäure, äquivalent durchzuführen für Phthalsäure/Isophthalsäure). Folgende Geräte wurden verwendet: Flüssigprobengeber, PAL Injektor 80; Gaschromatograph Agilent GC 7890 und Massenspektrometer Agilent MSD 5975.

Die Messungen erfolgten mit folgenden Parametern:

Kapillarsäule: 30 m DB-5; 0,25 mm Innendurchmesser; 0,25 μm Film
Trägergas: Helium
Säulenfluss: ca. 1 ml/min
Injektor: 300°C (0,5 min splitlos, dann 50 ml/min)
Ofentemperatur: 60°C - 8°C/min - 250°C (15 min)
Injektionsvolumen: 1,0 μl
Detektor (MSD): Single-Ion-Monitoring (SIM)
Gruppe 1 (6 min bis12 min): (91,0; 100 msec), (119,0; 100 msec), (150; 100 msec)
Gruppe 2 (ab 12 min): (163,0; 100 msec), (194,0; 100 msec)

Als Reagenzien wurden eingesetzt:

Derivatisierungsmittel Diazomethan
Methanol
Dotierlösung Terephthalsäure (Konzentration = 1 mg/ml, gelöst in Methanol)

**Probenvorbereitung**

**Derivatisierung der Probe**

25 mg Probe wurden in 1 ml Methanol gelöst und mit Diazomethan derivatisiert. Das Reaktionsgemisch wird danach im Wasserbad bis auf 1,5 ml eingeengt. Die gesamte Lösung wird in einen 2 ml-Messkolben überführt und bis zur Markierung mit Methanol aufgefüllt.

**Derivatisierung der mit Terephthalsäure dotierten Probe**

25 mg Probe wurden in 1 ml Methanol gelöst, mit 10 μL der Dotierlösung Terephthalsäure versetzt und im Anschluss mit Diazomethan derivatisiert. Das Reaktionsgemisch wird danach im Wasserbad bis auf 1,5 ml eingeengt. Die gesamte Lösung wird in einen 2 ml-Messkolben überführt und bis zur Markierung mit Methanol aufgefüllt.

**Auswertung**

Der Nachweis der Terephthalsäure erfolgte in der GC/MS-Analyse als Dimethylterephthalat. Der Dimethylterephthalat-Peak wird anhand der Laufzeit und anhand des Verhältnisses der beiden gemessenen Ionen m/z 163 und m/z 194 (zwei charakteristische Ionen für Dimethylterephthalat) identifiziert. Zur Berechnung des Massengehaltes an Terephthalsäure in der Probe werden die Messwerte aus der Messung der undotierten und aus der dotierten Probe herangezogen werden:

$$w\,T \;=\; \frac{(m,T\,dot.)}{\left(\left(\dfrac{(A(T,dot.)\;\times\;E}{E\,(dot.)\;\times\;A(T)}\right)-1\right)\;\times\;E(dot.)}$$

w(T): Massenanteil an Terephthalsäure in der Probe (in mg/g)
m(T, dot.): Masse an dotierter Terephthalsäure in der dotierten Probe (in μg)
A(T, dot.): Peakfläche des Dimethylterephthalat in der dotierten Probe
E(dot.): Einwaage Probe der dotierten Messung (in mg)
A(T): Peakfläche an Dimethylterephthalat in der undotierten Probe
E: Einwaage Probe der undotierten Messung (in mg)

**Apparatur:**

Als Apparatur wurde ein Quenchturm mit einem Volumen von 300 m$^3$ und einer inneren Höhe von 35,05 m eingesetzt. In der Apparatur waren in einer inneren Höhe von 3,96 bis 8,102 m 19 Packungen vom Typ Montz-Pak B1-125,60, in einer inneren Höhe von 10,975 bis 19,585 m 42 Packungen vom Typ Montz-Pak B1-250 Packungen und in einer inneren Höhe von 21,97 bis 28,53 m 32 Packungen vom Typ Montz-Pak B1-250 der Firma Montz installiert. Der Zulauf für das Quenchmittel war bei einer inneren Höhe von 32,598 m installiert, die Zuführung des Reaktionsgemisches erfolgte bei einer inneren Höhe von 5,048 m. Im Vergleichsbeispiel wurde ein solcher Quenchturm, der keinen Siebboden aufwies, eingesetzt. Der Quenchturm wies bei einer inneren Höhe von 28,3 m einen Siebboden auf, mit dem sich eine flüssige Schicht von 3 cm realisieren ließ. Die oberste Packung wurde verkleinert, so dass sie von einer inneren Höhe von 21,97 bis 27,3 m reichte. Der Quenchturm wies des weiteren Flüssigkeitsverteiler, Gasverteiler, Sammler sowie übliche Auflageroste für die Packungen auf. Am Kopf des Quenchturms war ein Kopfabzug vorhanden und im Sumpf der Kolonne ein Sumpfablauf.

**Vergleichsbeispiel 1: Herstellung Acrylsäure ohne die Verwendung einer erfindungsgemäßen Quenchturms**

In einer wie in Fig. 1 schematisch dargestellten Anlage zur Herstellung von Acrylsäure wurde ein Reaktionsgemischstrom, der 6,24 Vol.-% Acrylsäure und einen Gehalt der Summe aus Phthalsäure, Isophthalsäure und Terephthalsäure von 0,0144 Gew.-% aufwies, mit 71141 kg/h in den Quenchturm geleitet. Über den Zulauf für das Quenchmittel wurden 8200 kg/h an Wasser zugeführt. Über den Sumpfablauf wurde ein Produktstrom von 18252 kg/h entnommen. Über den Kopfabzug wurde ein Abgasstrom von 61089 kg/h entnommen. Die Sumpftemperatur wurde auf 79,1 °C und die Kopftemperatur auf 71,6 °C eingestellt. Nach dreistündigem kontinuierlichem Betrieb wurden die Proben aus dem Sumpf gezogen. Die Ergebnisse der Analyse sind in Tabelle 1 angegeben.

Die Anlage musste nach 33 Betriebstagen abgestellt werden, da der Druckverlust zwischen Reaktionsgemischzuführung und Kopfabzug um mehr als 50 % gegenüber dem Wert beim Anfahren der Anlage gestiegen war.

**Beispiel 1: Herstellung von Acrylsäure unter Verwendung eines erfindungsgemäßen Quenchturms.**

In einer wie im Vergleichsbeispiel beschriebenen Anlage wurde an Stelle des dort beschriebenen Quenchturms ein Quenchturm eingesetzt, der einen Siebboden aufwies. Nach dreistündigem kontinuierlichem Betrieb wurden die Proben aus dem Sumpf gezogen. Die Ergebnisse der Analyse sind wiederum in Tabelle 1 angegeben.

Im Gegensatz zum Vergleichsversuch konnte die Herstellung der Acrylsäure auch nach 2 Monaten noch problemlos durchgeführt werden. Bei einer standardmäßigen Revision der Anlage nach 3 Monaten konnten im Kopf des Quenchturms keinerlei Ablagerungen beobachtet werden, die auf Phthalsäure, Terephthalsäure oder Isophthalsäure oder Folgeprodukten davon, zurückgeführt werden können.

**Vergleichsbeispiel 2: Quenchturm ohne Packungen und ohne Siebboden:**

Das Vergleichsbeispiel 1 wurde wiederholt, wobei ein Quenchturm eingesetzt wurde, der statt der B1-250 Packungen 25 Thormann-Böden und darunter an Stelle der B1-125,60 Packungen 7 Segmentkaskadenböden aufwies.

Tabelle 1: Gehalt an Disäuren im Sumpfprodukt bezogen auf die Gesamtzusammensetzung des Sumpfproduktes

| | Summe Phthalsäure und Terephthalsäure | Isophthalsäure | Durchsatz Reaktionsgemischstrom |
|---|---|---|---|
| Vergleichsbeispiel 1 | 0,016 Gew.-% | 0,026 Gew.-% | 71141 kg/h |
| Beispiel 1 | 0,030 Gew.-% | 0,026 Gew.-% | 71141 kg/h |
| Vergleichsbeispiel 2 | 0,028 Gew.-% | 0,026 Gew.-% | 51100 kg/h |

Wie der Tabelle 1 entnommen werden kann, weist das Sumpfprodukt, welches in einer Apparatus mit einem Siebboden (Beispiel 1) erhalten wurde, einen Anteil an Phthalsäure und Terephthalsäure auf, der deutlich höher liegt als der Anteil, der für das Vergleichsbeispiel 1 bestimmt wurde. Mit einem Quenchturm ohne Packungen (Vergleichsbeispiel 2) können zwar ähnliche Anteile an Phthalsäure und Terephthalsäure erreicht werden, wie für die Apparatur mit Siebboden angegeben, allerdings nur unter Hinnahme von einer deutlichen Verringerung beim Durchsatz.

**Patentansprüche**

1. Verfahren zur Herstellung von Acrylsäure, welches einen Schritt der Oxidation von Acrolein aufweist, wobei das bei dieser Oxidation erhaltene Reaktionsgemisch in einer Apparatur mit Wasser als Quenchmittel in Kontakt gebracht wird, und wobei die Apparatur die folgenden Merkmale umfasst:
eine Zuführung die unterhalb der Mitte der Apparatur angeordnet ist, einen Quenchmittelzulauf, der oberhalb der Zuführung angeordnet ist, einen Ablauf, der unterhalb der Zuführung angeordnet ist, einen Abzug, der oberhalb des Quenchmittelzulaufs angeordnet ist, wobei die Apparatur zwischen Quenchmittelzulauf und Zuführung einen Bereich aufweist, der mit einer oder mehreren Packungen versehen ist, wobei zwischen dem Quenchmittelzulauf und einer zwischen Quenchmittelzulauf und Zuführung vorhandenen Packung, mindestens ein Mittel vorhanden ist, welches eine flüssige Schicht mit einer Höhe von mindestens 1 cm ausbildet, wobei die flüssige Schicht in der Apparatur so angeordnet ist, dass gasförmige Stoffe, die durch die Zuführung in die Apparatur gelangen, durch die flüssige Schicht hindurchtreten, um in den Kopf der Apparatur zu gelangen, wobei das Mittel ein Wellenboden oder ein Siebboden ist.

2. Verfahren nach Anspruch 1, **dadurch gekennzeichnet, dass** die flüssige Schicht eine Höhe von 2 bis 10 cm, vorzugsweise von 3 bis 8 cm aufweist.

**3.** Verfahren nach zumindest einem der Ansprüche 1 bis 2, **dadurch gekennzeichnet, dass** die Packung eine strukturierte Packung oder eine Gewebepackung, bevorzugt eine strukturierte Packung ist.

**4.** Verfahren nach zumindest einem der Ansprüche 1 bis 3, **dadurch gekennzeichnet, dass** die Packung eine Höhe aufweist, die 50 bis 85 % der inneren Höhe der Apparatur beträgt.

**5.** Verfahren nach zumindest einem der Ansprüche 1 bis 4, **dadurch gekennzeichnet, dass** 1 bis 5, bevorzugt 2 oder 3, besonders bevorzugt 3 der Mittel zwischen dem Quenchmittelzulauf und einer zwischen Quenchmittelzulauf und Zuführung vorhandenen Packung vorhanden sind.

**6.** Verfahren nach zumindest einem der Ansprüche 1 bis 5, **dadurch gekennzeichnet, dass** zwischen dem untersten Mittel und der obersten Packung ein Verteiler vorhanden ist, der die vom untersten Mittel abfließende flüssige Phase auf der Packung möglichst gleichmäßig verteilt.

**7.** Verfahren nach Anspruch 6, **dadurch gekennzeichnet, dass** zwischen unterstem Mittel und Verteiler ein Sammler angeordnet ist, der die vom untersten Mittel abfließende flüssige Phase sammelt und dem Verteiler zuführt.

**8.** Verfahren nach Anspruch 1, **dadurch gekennzeichnet, dass** das Verhältnis der Volumenströme von Quenchmittel, welches der Apparatur zugeführt wird, zu Reaktionsgemisch, welches der Apparatur zugeführt wird, von 1 zu 2 bis 1 zu 20, bevorzugt von 1 zu 5 bis 1 zu 10 beträgt.

**9.** Verfahren nach Anspruch 1 oder 8, **dadurch gekennzeichnet, dass** die Temperatur der flüssigen Phase auf dem Mittel von 50 °C bis 100 °C, bevorzugt von 70 °C bis 80 °C beträgt.

**10.** Verfahren nach zumindest einem der Ansprüche 1 oder 8 oder 9, **dadurch gekennzeichnet, dass** die Apparatur so betrieben wird, dass die Druckdifferenz zwischen dem Druck, gemessen am Kopf der Apparatur und dem Druck, gemessen im Gasraum oberhalb der flüssigen Phase im Sumpf der Apparatur von 10 mbar bis 60 mbar, bevorzugt von 30 mbar bis 40 mbar beträgt.

**11.** Verfahren nach zumindest einem der Ansprüche 1 oder 8 oder 9 oder 10, **dadurch gekennzeichnet, dass** der Apparatur pro Stunde von 210 bis 380 kg Reaktionsgemisch pro m$^3$ Apparaturvolumen zugeführt wird.

**Claims**

**1.** Process for preparing acrylic acid, including a step of oxidizing acrolein, wherein the reaction mixture obtained in this oxidation is contacted with water as quenching agent in an apparatus, and wherein the apparatus comprises the following features:
a feed arranged below the middle of the apparatus, a quenching agent inlet arranged above the feed, an outlet arranged below the feed and a draw arranged above the quenching agent inlet, wherein the apparatus has, between the quenching agent inlet and the feed, a region provided with one or more packing elements, wherein at least one means which forms a liquid layer having a height of at least 1 cm is present between the quenching agent inlet and a packing element present between the quenching agent inlet and feed, wherein the liquid layer is arranged in the apparatus such that gaseous substances which pass through the feed into the apparatus pass through the liquid layer in order to get into the top of the apparatus, wherein the means is a ripple tray or a sieve tray.

**2.** Process according to Claim 1, **characterized in that** the liquid layer has a height of 2 to 10 cm, preferably of 3 to 8 cm.

**3.** Process according to at least one of Claims 1 and 2, **characterized in that** the packing element is a structured packing element or a fabric packing element, preferably a structured packing element.

**4.** Process according to at least one of Claims 1 to 3, **characterized in that** the packing element has a height of 50 to 85% of the internal height of the apparatus.

**5.** Process according to at least one of Claims 1 to 4, **characterized in that** 1 to 5, preferably 2 or 3, more preferably 3, of the means are present between the quenching agent inlet and a packing element present between the quenching agent inlet and feed.

6. Process according to at least one of Claims 1 to 5, **characterized in that** a distributor present between the lowermost means and the uppermost packing element distributes the liquid phase flowing downward from the lowermost means with maximum homogeneity over the packing element.

7. Process according to Claim 6, **characterized in that** a collector arranged between the lowermost means and distributor collects the liquid phase flowing downward from the lowermost means and feeds it to the distributor.

8. Process according to Claim 1, **characterized in that** the ratio of the volume flow rates of quenching agent which is fed to the apparatus to the reaction mixture which is fed to the apparatus is from 1:2 to 1:20, preferably from 1:5 to 1:10.

9. Process according to Claim 1 or 8, **characterized in that** the temperature of the liquid phase on the means is from 50°C to 100°C, preferably from 70°C to 80°C.

10. Process according to at least one of Claims 1 and 8 and 9, **characterized in that** the apparatus is operated in such a way that the pressure differential between the pressure measured at the top of the apparatus and the pressure measured in the gas space of the liquid phase in the bottom of the apparatus is from 10 mbar to 60 mbar, preferably from 30 mbar to 40 mbar.

11. Process according to at least one of Claims 1 and 8 and 9 and 10, **characterized in that** the apparatus is fed with 210 to 380 kg of reaction mixture per hour per $m^3$ of apparatus volume.

**Revendications**

1. Procédé pour la préparation d'acide acrylique, lequel comprend une étape d'oxydation d'acroléine, le mélange de réaction obtenu lors de cette oxydation étant amené en contact dans un appareil avec de l'eau en tant qu'agent de trempe, et l'appareil comprenant les caractéristiques suivantes :
une alimentation, qui est disposée au-dessous du centre de l'appareil, une arrivée d'agent de trempe, qui est disposée au-dessous de l'alimentation, un écoulement, qui est disposé au-dessous de l'alimentation, une évacuation, qui est disposée au-dessus de l'arrivée d'agent de trempe, l'appareil possédant une zone entre l'arrivée d'agent de trempe et l'alimentation, laquelle est pourvue d'une ou de plusieurs garnitures, au moins un moyen étant présent entre l'arrivée d'agent de trempe et une garniture présente entre l'arrivée d'agent de trempe et l'alimentation, lequel forme une couche liquide ayant une hauteur d'au moins 1 cm, la couche liquide étant disposée dans l'appareil de telle sorte que des substances gazeuses qui parviennent dans l'appareil par le biais de l'alimentation passent à travers la couche liquide afin de parvenir dans la tête de l'appareil, le moyen étant un fond ondulé ou un fond à tamis.

2. Procédé selon la revendication 1, **caractérisé en ce que** la couche liquide présente une hauteur de 2 à 10 cm, de préférence de 3 à 8 cm.

3. Procédé selon au moins l'une des revendications 1 et 2, **caractérisé en ce que** la garniture est une garniture structurée ou une garniture tissée, de préférence une garniture structurée.

4. Procédé selon au moins l'une des revendications 1 à 3, **caractérisé en ce que** la garniture présente une hauteur qui est de 50 à 85 % de la hauteur interne de l'appareil.

5. Procédé selon au moins l'une des revendications 1 à 4, **caractérisé en ce que** 1 à 5, préférablement 2 ou 3, particulièrement préférablement 3 des moyens sont présents entre l'arrivée d'agent de trempe et une garniture présente entre l'arrivée d'agent de trempe et l'alimentation.

6. Procédé selon au moins l'une des revendications 1 à 5, **caractérisé en ce qu'**entre le moyen le plus bas et la garniture la plus haute se trouve un répartiteur, qui répartit de manière la plus homogène possible sur la garniture, la phase liquide qui s'écoule depuis le moyen le plus bas.

7. Procédé selon la revendication 6, **caractérisé en ce qu'**entre le moyen le plus bas et le répartiteur est disposé un collecteur qui collecte la phase liquide qui s'écoule depuis le moyen le plus bas et l'alimente au répartiteur.

8. Procédé selon la revendication 1, **caractérisé en ce que** le rapport des débits volumiques entre l'agent de trempe qui est alimenté à l'appareil et le mélange de réaction qui est alimentée à l'appareil est de 1 sur 2 à 1 sur 20, de préférence de 1 sur 5 à 1 sur 10.

9. Procédé selon la revendication 1 ou 8, **caractérisé en ce que** la température de la phase liquide sur le milieu est de 50 °C à 100 °C, de préférence de 70 °C à 80 °C.

10. Procédé selon au moins l'une des revendications 1 ou 8 ou 9, **caractérisé en ce que** l'appareil fonctionne de telle sorte que la différence de pression entre la pression mesurée au niveau de la tête de l'appareil et la pression mesurée dans l'espace à gaz au-dessus de la phase gazeuse dans le fond de l'appareil est de 10 mbars à 60 mbars, préférablement de 30 mbars à 40 mbars.

11. Procédé selon au moins l'une des revendications 1 ou 8 ou 9 ou 10, **caractérisé en ce que** de 210 à 380 kg de mélange de réaction par m$^3$ de volume d'appareil par heure sont alimentés à l'appareil.

Fig. 1

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1319648 A1
- DE 4308087
- DE 102010028781 A1
- US 7189872 B
- EP 0982289 A2
- US 6293528 B1
- DE 19740252
- EP 0092097 A
- EP 0058927 A
- EP 0608838 A